# EUROPÄISCHE PATENTSCHRIFT

(11) **EP 1 320 757 B1**
(45) Veröffentlichungstag und Bekanntmachung des Hinweises auf die Patenterteilung: **30.11.2005**
(21) Anmeldenummer: 01972068.9
(22) Anmeldetag: 18.09.2001
(51) Int. Cl.: G01N 33/66

(54) **VERFAHREN ZUR DIAGNOSE DER LAKTOSE-INTOLERANZ UND DIAGNOSEKIT ZUR DURCHFÜHRUNG DES VERFAHRENS**
METHOD FOR DIAGNOSING LACTOSE INTOLERANCE AND DIAGNOSIS KIT FOR CARRYING OUT THE METHOD
PROCEDE POUR DIAGNOSTIQUER L'INTOLERANCE AU LACTOSE ET TROUSSE DE DIAGNOSTIC POUR REALISER CE PROCEDE

(30) Priorität: 27.09.2000 DE 10060989
(43) Veröffentlichungstag der Anmeldung: 25.06.2003
(73) Patentinhaber: Aygen, Sitke, 51105 Köln (DE)
(72) Erfinder: Aygen, Sitke, 51105 Köln (DE)
(74) Vertreter: Schreiber, Christoph
(86) Internationale Anmeldenummer: PCT/EP2001/010767
(87) Internationale Veröffentlichungsnummer: WO 2002/027325

(56) Entgegenhaltungen:
- US-A- 5 640 014
- PEUHKURI K ET AL: "Wide variations in the testing of lactose tolerance: Results of a questionnaire study in Finnish health care centres." SCANDINAVIAN JOURNAL OF CLINICAL AND LABORATORY INVESTIGATION, Bd. 60, Nr. 4, Juli 2000 (2000-07), Seiten 291-298, XP001027414 ISSN: 0036-5513
- HIELE M ET AL: "CARBON-13 DIOXIDE BREATH TEST USING NATURALLY CARBON-13-ENRICHED LACTOSE FOR DETECTION OF LACTASE DEFICIENCY IN PATIENTS WITH GASTROINTESTINAL SYMPTOMS" JOURNAL OF LABORATORY AND CLINICAL MEDICINE, Bd. 112, Nr. 2, 1988, Seiten 193-200, XP001027178 ISSN: 0022-2143
- MURRAY R D ET AL: "COMPARATIVE ABSORPTION OF CARBON-13 GLUCOSE AND CARBON-13 LACTOSE BY PREMATURE INFANTS" AMERICAN JOURNAL OF CLINICAL NUTRITION, Bd. 51, Nr. 1, 1990, Seiten 59-66, XP001027415 ISSN: 0002-9165
- HERMANS MARC M H ET AL: "The relationship between lactose tolerance test results and symptoms of lactose intolerance." AMERICAN JOURNAL OF GASTROENTEROLOGY, Bd. 92, Nr. 6, 1997, Seiten 981-984, XP001027136 ISSN: 0002-9270
- VONK ROEL J ET AL: "Small intestinal and colonic factors involved in lactose intolerance." FASEB JOURNAL, Bd. 15, Nr. 4, 7. März 2001 (2001-03-07), Seite A277 XP001027262 Annual Meeting of the Federation of American Societies for Experimental Biology on Experimental Biology 2001;Orlando, Florida, USA; March 31-April 04, 2001 ISSN: 0892-6638
- VONK R J ET AL: "THE 13C/2H-GLUCOSE TEST FOR DETERMINATION OF SMALL INTESTINAL LACTASE ACTIVITY" EUROPEAN JOURNAL OF CLINICAL INVESTIGATION, BLACKWELL SCIENTIFIC PUBLICATIONS, XX, Bd. 31, Nr. 3, 2001, Seiten 226-233, XP000998471 ISSN: 0014-2972

## Beschreibung

Gegenstand der vorliegenden Erfindung ist ein Verfahren zur Diagnose der Laktose-Intoleranz von Patienten durch orale Applikation von definierten Mengen Laktose und anschließender Bestimmung von Stoffwechselprodukten der Laktose sowie einem. Diagnosekit zur Durchführung des Verfahrens.

Lactose-Intoleranz ist eine weltweit sehr häufige Erscheinung und führt bei den Betreffenden zu einer mehr oder weniger starken Verdauungsstörung durch Milch und Milchprodukte. Die Lacose-Intoleranz beruht im allgemeinen auf dem Fehlen des Enzyms Laktase, für dessen Bildung ein Gen auf dem Chromosom 2 verantwortlich ist. Dieses Gen bestimmt den Zeitpunkt, zu dem der Mensch keine Laktase mehr produziert. Je nach ethnischer Zugehörigkeit kann das früher oder später der Fall sein. In früheren Zeiten der Entwicklungsgeschichte verlor der Mensch wie viele andere Säugetiere das Enzym stets dann, wenn er nicht mehr von der Muttermilch genährt wurde (vgl. Pharm. Ztg. Nr. 9 · 145. Jahrgang · 2. März 2000). Für die Diagnostik der Laktose-Intoleranz wird heute am häufigsten da einfach und aussagekräftig der H₂-Atemtest eingesetzt. Dazu trinkt der Patient eine Lösung von 50 g Laktose in Wasser. Der anschließend ausgeatmete Wasserstoff wird dann gaschromatographisch über 4 Stunden wiederholt gemessen. Auf diese Weise erhält man nicht nur eine qualitative, sondern auch eine quantitative Aussage über den Laktasemangel.

Die Lactose-Intoleranz wird inzwischen als Krankheit angesehen, die bislang nur symptomatisch zu behandeln ist. Es gibt jedoch inzwischen die Möglichkeit, das Enzym Laktase in Form von Kapseln, Tabletten oder Lösung einzunehmen und dadurch das Defizit an Laktase zu kompensieren.

Außer dem bereits erwähnten Wasserstoff in der Atemluft gibt es noch einen Laktose-Toleranz-Test, bei dem nüchternen Testpersonen 50 g Laktose in Wasser appliziert wird und dann bei dieser Person über mehrere Stunden hinweg der Blut-Glukose-Spiegel gemessen wird. Sofern die Laktose nicht vollständig enzymatisch gespalten wird, bleibt der Glukose-Spiegel niedrig und bestätigt somit die Laktose-Intoleranz, vgl. Scand. J. Clin. Lab 2000, 60 S. 291-296.

Die bisher bekannten Methoden der Diagnose weisen den Nachteil auf, dass man den Patienten eine relativ große Menge an Laktose zuführen muss, was bei vielen Patienten, die unter Laktose-Intoleranz leiden, bereits zu erheblichen Beschwerden führen kann.

Die Bestimmung der Blut-Glukose weist den weiteren Nachteil auf, dass der Blut-Glukose-Spiegel durch sekundäre Einflüsse verändert werden kann, beispielsweise erhöhte Adrenalinausschüttung durch Stress.

Beide Methoden des Standes der Technik weisen weiterhin den Nachteil auf, dass die Messung mit mehreren Proben über einen längeren Zeitraum hin durchgeführt werden müssen, was für den Patienten unangenehm und belastend ist und die Kosten in die Höhe treibt.

Theoretisch bestünde die Möglichkeit, den Test mit ¹³C-markierter Laktose durchzuführen. Ein Gramm ¹³C-markierte Laktose kostet aber US $ 2000. Für den Laktose Atemtest werden mindestens 150 mg 99% ¹³C-markierte Laktose benötigt.

Die Erfindung hat sich die Aufgabe gestellt, ein besseres, preiswerteres und genaueres Verfahren zur Diagnose der Laktose-Intoleranz zur Verfügung zu stellen, welches die oben genannten Nachteile nicht aufweist.

Diese Aufgabe wird erfindungsgemäß gelöst durch orale Applikation von definierten Mengen Laktose und anschließender Bestimmung von Stoffwechselprodukten der Laktose, wobei die applizierte Laktose 99% ¹³C-markierte Laktose enthält, und zwar in Mengen von 5 bis 30 mg. Nach der Applikation der ¹³C-Laktose wird in einer zu einem definierten Zeitpunkt nach der Applikation entnommenen Blutprobe der Gehalt an ¹³C-markierter Glukose bestimmt. Vorzugsweise wird die ¹³C-Laktose in Form einer Gelatinekapsel appliziert. Hierdurch ist es möglich, dass die ¹³C-Laktose in den Dünndarm gelangt ohne im Magen absorbiert zu werden.

Das Verfahren kann darüber hinaus dadurch bezüglich der Aussagekraft verbessert werden, indem zusätzlich in einer einige Zeit nach dem Zeitpunkt der Blutentnahme gewonnenen Stuhlprobe der Gehalt an ¹³C-markierter Laktose und/oder ¹³C-markierter Milchsäure bestimmt wird. Diese zusätzliche Bestimmung ist nur dann notwendig, wenn die Untersuchung der Serumprobe auf Glukose keinen signifikanten Anstieg an ¹³C zeigt und somit die Laktose-Intoleranz eigentlich schon feststeht.

Patienten mit sehr stark ausgeprägter Laktose-Intoleranz können bereits durch die reduzierte Menge von 5 bis 10 g Laktose unter Diarrhöe leiden. Derartige Stuhlproben enthalten dann noch nahezu die Gesamtmenge an ¹³C-markierter Laktose. Bei Patienten mit weniger stark ausgeprägter Laktose-Intoleranz ermöglicht es den Darmbakterien, die applizierte Laktose ganz oder teilweise zu ¹³C-markierter Milchsäure zu verstoffwechseln. Darüber hinaus kann in solchen Fällen auch ¹³C-markiertes Methan und H₂ entstehen, die jedoch wesentlich schwerer zu untersuchen sind. Die Absenkung der pH-Werte der Stuhlproben durch Milchsäure ist ein weiteres Indiz für Laktose-Intoleranz. Der pH-Wert des Stuhls kann aber durch viele andere Faktoren beeinflusst werden, so dass dieser Parameter kaum geeignet ist, Laktose-Intoleranz sicher zu diagnostizieren.

Das erfindungsgemäße Verfahren kommt im Gegensatz zu den bisher verwendeten diagnostischen Verfahren mit einer deutlich geringeren Menge von Laktose aus, nämlich 5 bis 30 mg 99% ¹³C-Laktose in einer Kapsel. Diese geringere Menge führt bei Patienten mit starker Laktose-Intoleranz zu keinen Beschwerden während des diagnostischen Verfahrens.

Von entscheidender Bedeutung ist aber, dass die erfindungsgemäße Methode wesentlich genauer ist und obendrein aussagekräftiger, da sie nicht durch sekundäre Einflüsse gestört wird. Dies gilt bereits für den H₂-Atemtest und in viel größerem Maße für die Bestimmung der Glukose im Serum.

Die erfindungsgemäße Bestimmung des ¹³C-Gehalts in den Serumproben und/oder den Stuhlproben erfolgt insbesondere mit Hilfe moderner Massenspektrographie mit vorgeschalteter Gaschromatographie oder Elementaranalyse. Diese Methoden sind inzwischen soweit weiterentwickelt und automatisierbar, dass es wirtschaftlich möglich ist, die Proben der Patienten an ein entsprechend ausgerüstetes Zentrallabor zu versenden und dort untersuchen zu lassen.

Das Zentrallaboratorium sollte in erster Linie nur die Serumproben analysieren. Sofern auch Stuhlproben mit eingesandt werden, sollten diese nur dann untersucht werden, wenn die entsprechende Serumprobe keinen signifikanten Anstieg der ¹³C-markierten Glukose ergeben hat. Bei normalem Anstieg des ¹³C-Gehaltes im Vergleich zu Standardproben mit bekanntem ¹³C-Gehalt wird bereits angezeigt, dass Laktose gut vertragen und ausreichend durch Laktase zu Glukose abgebaut wird. Nur bei keinem oder deutlich geringerem Anstieg des ¹³C-Gehaltes kann durch Untersuchung der Stuhlprobe festgestellt werden, ob es sich um eine sehr starke oder schwächer ausgebildete Laktose-Intoleranz handelt.

Sofern der Test einen normalen Anstieg des ¹³C-Gehaltes ergibt und dennoch Milch und Milchprodukte schlecht vertragen werden, handelt es sich um andere Erkrankungen als die weit verbreitete Laktose-Intoleranz. Das erfindungsgemäße Verfahren kann somit auch differentialdiagnostisch eingesetzt werden. Dieser Gruppe von Patienten kann dann nicht durch Einnahme von Laktase-Präparaten geholfen werden.

Das erfindungsgemäße Verfahren ist durch das nachfolgende Beispiel näher erläutert.

### Beispiel

Vor Testbeginn wird dem nüchternen Probanden 0,3 bis 0,5 ml Kapillarblut aus dem Finger oder Ohrläppchen entnommen. Dann nimmt der Proband eine Gelatinekapsel mit 5 bis 30 mg ¹³C-markierter Laktose ein. 30 bis 60 min nach der Einnahme der markierten Laktose werden erneut 0,3 bis 0,5 ml Blut, wie oben beschrieben, entnommen. Aus den Blutproben wird durch Zentrifugation Serum gewonnen. Mittels Filter werden aus den Serumsproben höhermolekulare Bestandteile (z.B. Lipide und Proteine) entfernt. Die Filtrate werden mittels einer Elementaranalyse verbrannt. Das dabei unter anderem aus Glucose gebildete CO₂ wird in ein Isotopen-Massenspektrometer geleitet, womit der ¹³C-Gehalt bestimmt wird. Wie oben beschrieben, werden beide Blutproben ausgewertet. Wenn zwischen den Blutproben vor und nach der Einnahme der ¹³C-Laktose einen Differenzwert Δδ von weniger als 1‰ nachgewiesen wird, liegt eine Laktoseintoleranz vor.

Der Text verläuft nach folgendem Schema:

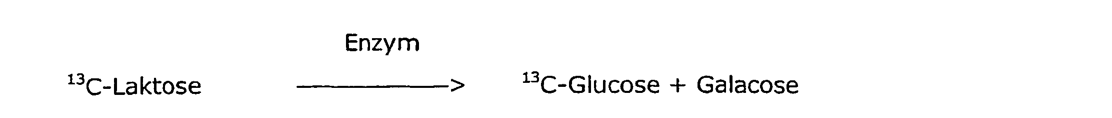

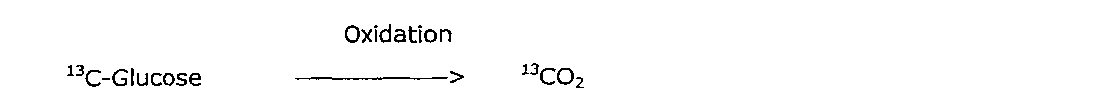

Der Patient entnimmt von dem nächsten Stuhl eine Probe und trägt sie mit dem Spachtel in ein Probengefäß. Der nächste Stuhl erfolgt im allgemeinen 2 bis 4 Stunden nach Einnahme der Kapsel. Aus den Stuhlproben wird im Laboratorium ein wässriger Extrakt hergestellt und dieser auf ¹³C-Laktose und/oder ¹³C-Milchsäure untersucht. Anwesenheit von ¹³C-Laktose bzw. ¹³C-Milchsäure bestätigt die vorliegende Laktose-Intoleranz.

## Patentansprüche

1. Verfahren zur Diagnose der Laktose-Intoleranz von Patienten durch orale Applikation von definierten Mengen Laktose und anschließender Bestimmung von Stoffwechselprodukten der Laktose, **dadurch gekennzeichnet, dass** die applizierte Laktose 99% ¹³C-markierte Laktose enthält und nach der Applikation der ¹³C-Laktose in einer zu einem definierten Zeitpunkt nach der Applikation entnommenen Serumprobe der Gehalt an ¹³C-markierter Glucose bestimmt wird, wobei die Menge an applizierter 99% ¹³C-Laktose 5 bis 30 mg beträgt

2. Verfahren gemäß Anspruch 1, **dadurch gekennzeichnet, dass** die ¹³C-markierte Laktose in einer Kapsel appliziert wird.

3. Verfahren gemäß Anspruch 1 oder 2, **dadurch gekennzeichnet, dass** zusätzlich bestimmt wird in einer einiger Zeit nach dem Zeitpunkt der Serumentnahme gewonnenen Stuhlprobe der Gehalt an ¹³C-markierter Laktose und/oder ¹³C-markierter Milchsäure.

4. Verfahren gemäß einem der Ansprüche 1 bis 3, **dadurch gekennzeichnet, dass** die Serumprobe ca. eine Stunde nach Applikation der Laktose entnommen wird.

5. Verfahren gemäß Anspruch 3 oder 4, **dadurch gekennzeichnet, dass** die Stuhlprobe 2 bis 4 Stunden nach der Einnahme der Kapsel entnommen wird.

6. Verfahren gemäß einem der Ansprüche 1 bis 5, **dadurch gekennzeichnet, dass** die Proben an ein Zentrallabor versandt werden und dort mittels Massenspektrometrie mit vorgeschalteter Gaschromatographie oder Elementaranalyse auf den ¹³C-Gehalt untersucht werden.

7. Diagnosekit zur Durchführung des Verfahrens gemäß einem der Ansprüche 1 bis 6 bestehend aus einer
1. 5 bis 30 mg 99% ¹³C-markierter Laktose in einer Kapsel,
2. einer Patienteninformation,
3. einer Blutentnahmevorrichtung,
4. einem Probegefäß zur Sammlung der. Blutprobe sowie gegebenenfalls
5. einem Probegefäß und Spachtel für eine Stuhlprobe.

## Claims

1. A method for diagnosing lactose intolerance in patients by the oral administration of defined amounts of lactose, followed by assaying metabolites of lactose, **characterized in that** the lactose administered contains 99% ¹³C-labeled lactose, and after the administration of the ¹³C-lactose, the content of ¹³C-labeled glucose is determined in a serum sample taken at a defined time after the administration, the amount of 99% ¹³C-lactose administered being from 5 to 30 mg.

2. The method according to claim 1, **characterized in that** said ¹³C-labeled lactose is administered in a capsule.

3. The method according to claim 1 or 2, **characterized in that** the content of ¹³C-labeled lactose and/or ¹³C-labeled lactic acid is additionally determined in a stool specimen obtained some time after the time of the serum sampling.

4. The method according to any of claims 1 to 3, **characterized in that** the serum sample is withdrawn about one hour after the administration of the lactose.

5. The method according to claim 3 or 4, **characterized in that** said stool specimen is taken 2 to 4 hours after the ingestion of the capsule.

6. The method according to any of claims 1 to 5, **characterized in that** the samples are sent to a central laboratory where they are examined for their ¹³C content by mass spectrography with previously performed gas chromatography or elemental analysis.

7. A diagnostic kit for performing the method according to any of claims 1 to 6, consisting of:
1) from 5 to 30 mg of 99% ¹³C-labeled lactose in a capsule;
2) a patient instruction manual;
3) a blood sampling device;
4) a sample container for collecting the blood sample; and optionally
5) a sample container and spatula for a stool specimen.

## Revendications

1. Méthode de diagnostic de l'intolérance au lactose des patients par administration orale de quantités définies de lactose et détermination suivante des métabolites du lactose, **caractérisée en ce que** le lactose administré contient 99 % de lactose marqué au ¹³C et qu'après l'administration de lactose marqué au ¹³C, on détermine la teneur en glucose marqué au ¹³C dans un échantillon de sérum prélevé après l'administration à un moment défini, la quantité de lactose marqué au ¹³C à 99 % administrée étant de 5 à 30 mg.

2. Méthode selon la revendication 1, **caractérisée en ce que** le lactose marqué au ¹³C est administré dans une capsule.

3. Méthode selon la revendication 1 ou 2, **caractérisée en ce qu'**on détermine en plus, un certain temps après le moment du prélèvement du sérum sur un échantillon de selles obtenu, la teneur en lactose marqué au ¹³C et/ou en acide lactique marqué au ¹³C.

4. Méthode selon une des revendications 1 à 3, **caractérisée en ce qu'**on prélève l'échantillon de sérum env. 1 heure après l'administration du lactose.

5. Méthode selon la revendication 3 ou 4, **caractérisée en ce qu'**on prélève l'échantillon de selles 2 à 4 heures après la prise de la capsule.

6. Méthode selon une des revendications 1 à 5, **caractérisée en ce que** les échantillons sont envoyés à un laboratoire central et que la teneur en ¹³C y est analysée par spectrométrie de masse couplée à une chromatographie en phase gazeuse en amont ou par analyse élémentaire.

7. Kit de diagnostic servant à exécuter la méthode selon une des revendications 1 à 6 comprenant :
1) 5 à 30 mg de lactose marqué au ¹³C à 99 % sous forme d'une capsule,
2) une note d'information destinée aux patients,
3) un dispositif de prélèvement de sang,
4) un récipient d'échantillon destiné à collecter l'échantillon de sang ainsi qu'éventuellement
5) un récipient d'échantillon et une spatule pour l'échantillon de selles.
